# EUROPÄISCHE PATENTSCHRIFT

(11) **EP 0 790 245 B1**
(45) Veröffentlichungstag und Bekanntmachung des Hinweises auf die Patenterteilung: **13.06.2001**
(21) Anmeldenummer: 97101597.9
(22) Anmeldetag: 03.02.1997
(51) Int. Cl.: C07D 333/34, A61K 31/38

(54) **Substituierte Thiophenylalkenylcarbonsäureguanidide, Verfahren zu ihrer Herstellung, ihre Verwendung als Medikament oder Diagnostikum sowie sie enthaltendes Medikament**
Substituted Thiophenylalkenylcarbonylguanidide, method for their preparation, their use as medicinal or diagnostic agent as well as medicament containing them
Guanidides d'acides alcénylcarboxiliques substituée par un groupe thiophényl, leur procédé de préparation, leur utilisation comme médicament ou agent diagnostique ainsi que médicament les contenant

(30) Priorität: 15.02.1996 DE 19605610
(43) Veröffentlichungstag der Anmeldung: 20.08.1997
(73) Patentinhaber: HOECHST AKTIENGESELLSCHAFT, 65929 Frankfurt am Main (DE)
(72) Erfinder: Schwark, Jan-Robert, Dr., 65929 Frankfurt (DE); Brendel, Joachim, Dr., 61118 Bad Vilbel (DE); Kleemann, Heinz-Werner, Dr., 65474 Bischofsheim (DE); Lang, Hans Jochen, Dr., 65719 Hofheim (DE); Weichert, Andreas, Dr., 63329 Egelsbach (DE); Albus, Udo, Dr., 61197 Florstadt (DE); Scholz, Wolfgang, Dr., 65760 Eschborn (DE)

(56) Entgegenhaltungen:
- EP-A- 0 676 395
- DE-A- 4 402 057
- DE-A- 4 421 536
- US-A- 2 734 904
- US-A- 4 544 670

## Beschreibung

Substituierte Thiophenylalkenylcarbonsäureguanidide, Verfahren zu ihrer Herstellung, ihre Verwendung als Medikament oder Diagnostikum sowie sie enthaltendes Medikament

Die Erfindung betrifft substituierte Thiophenylalkenylcarbonsäureguanidide der Formel I 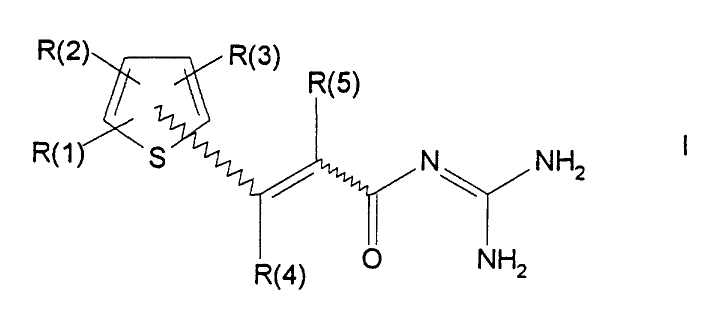 worin bedeuten:
mindestens einer der Substituenten R(1), R(2) und R(3)
   R(31)SOₖ-;
   - k: Null oder 2;
   - R(31): CH₃, CF₃ oder Phenyl,
   das nicht substituiert oder substituiert ist mit 1 - 3 Substituenten ausgewählt aus der Gruppe bestehend aus F, Cl, CF₃, Methyl und Methoxy;
   oder
   - R(31) NR(41)R(42);: R(41) und R(42) unabhängig voneinander Wasserstoff, CH₃ oder CF₃; oder
   R(41) und R(42) gemeinsam 4 oder 5 Methylengruppen, von denen eine CH₂-Gruppe durch Sauerstoff, S, NH, N-CH₃ oder N-Benzyl ersetzt sein kann;
   und die jeweils anderen Substituenten R(1), R(2) und R(3)
   unabhängig voneinander H, F, Cl, CN, -Oₙₐ-CₘₐH₂ₘₐ₊₁ oder -O_{ga}CᵣₐH₂ᵣₐR(10);
   - na: Null oder 1;
   - ma: Null, 1, 2, 3 oder 4;
   - ga: Null oder 1;
   - ra: Null oder 1;
   - R(10): Phenyl,
   das nicht substituiert oder substituiert ist mit 1 - 3 Substituenten ausgewählt aus der Gruppe bestehend aus F, Cl, CF₃, Methyl und Methoxy;
R(4) und R(5)
   unabhängig voneinander Wasserstoff, F, Cl, CH₃, CF₃; sowie deren pharmazeutisch verträgliche Salze.

Speziell besonders bevorzugt sind die Verbindungen:
E-3-[2-(4-Methylsulfonyl-thiophenyl)]propensäureguanidid
E-3-[2-(5-Methylthio-thiophenyl)]-2-methyl-propensäureguanidid
E-3-[2-(5-Methylsulfonyl-thiophenyl)]-2-methyl-propensäureguanidid
E-3-[2-(3-Chlor-4-isopropylsulfonyl-5-methylthio-thiophenyl)]-2-methyl-propensäureguanidid
E-3-[2-(3-Chlor-4-isopropylsulfonyl-5-methylsulfonyl-thiophenyl)]-2-methyl-propensäureguanidid
sowie deren pharmazeutisch verträgliche Salze.

Enthalten die Verbindungen der Formel I ein oder mehrere Asymmetriezentren, so können diese sowohl S als auch R konfiguriert sein. Die Verbindungen können als optische Isomere, als Diastereomere, als Racemate oder als Gemische derselben vorliegen.

Die Doppelbindungsgeometrie der Verbindungen der Formel I kann sowohl E als auch Z sein. Die Verbindungen können als Doppelbindungs-lsomere im Gemisch vorliegen.

Die bezeichneten Alkylreste und Perfluoralkylreste können sowohl geradkettig als auch verzweigt vorliegen.

Die Erfindung betrifft weiterhin ein Verfahren zur Herstellung der Verbindung I, dadurch gekennzeichnet, daß man eine Verbindung der Formel II 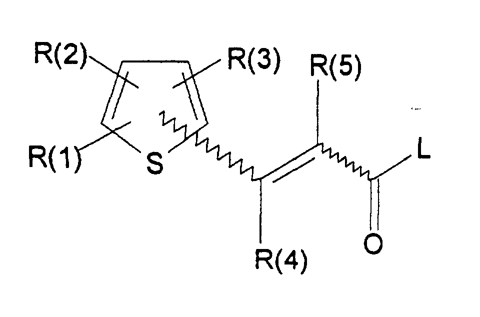 mit Guanidin umsetzt, worin R(1) bis R(5) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäurederivaten (Formel II, L = OH) herstellen, wie beispielsweise die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluoborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von 11 mit Guanidin verwendet werden.

Wenn L = Cl bedeutet, arbeitet man vorteilhaft unter Zusatz eines Säurefängers, z. B. in Form von überschüssigem Guanidin zur Abbindung der Halogenwasserstoffsäure.

Ein Teil der zugrundeliegenden Propensäurederivate der Formel II sind bekannt und in der Literatur beschrieben. Die unbekannten Verbindungen der Formel II können nach literaturbekannten Methoden hergestellt werden. Die erhaltenen Alkenylcarbonsäuren werden nach einer der oben beschriebenen Verfahrensvarianten zu erfindungsgemäßen Verbindungen I umgesetzt.

Die Einführung einiger Substituenten gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. zink-verbindungen.

Carbonsäureguanidide I sind im allgemeinen schwache Basen und können Säure unter Bildung von Salzen binden. Als Säureadditionssalze kommen Salze aller pharmakologisch verträglichen Säuren in frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 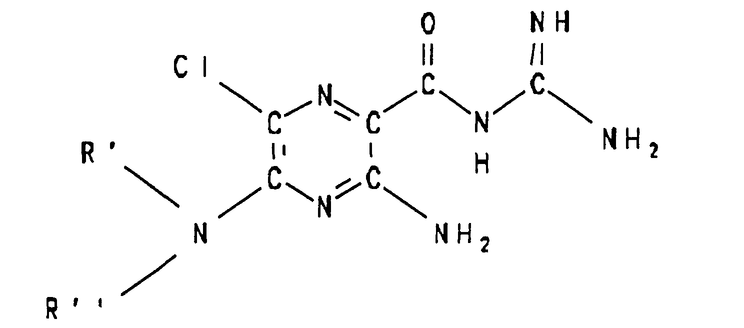
Amilorid: R',R'' = H
Dimethylamilorid: R',R'' = CH₃
Ethylisopropylamilorid: R' = C₂H₅, R'' = CH(CH₃)₂

Darüber hinaus sind Untersuchungen bekannt geworden, die auf antiarrhythmische Eigenschaften von Amilorid hinweisen (Circulation 79, 1257-63 (1989)). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

Aus WO 84/00875 sind Zimtsäureguanidide bekannt (Rₐ und R_{c}, bzw. R_{b} und R_{d} = Doppelbindung; R(1) = substituiertes Phenyl); jedoch sind darin Thiophen-Verbindungen weder beschrieben noch werden sie nahegelegt.

Aus der US-Patentschrift 2 734 904 sind Zimtsäureguanidide bekannt (R = substituiertes Phenyl, Alkyl = Alkenylen), jedoch werden keine Thiophen-Verbindungen der beanspruchten Art beschrieben oder nahegelegt. Bekannt sind daraus zwar Thiophen-alkenyl-carbonsäureguanidide, die aber nicht die wesentlichen Substituenten -Oₚ-(CH₂)ₛ-C_{q}F_{2q+1}, R(40)CO- oder R(31)SOₖ-tragen.

In der DE-OS 44 21 536.3 (HOE 94/F 168) werden Zimtsäureguanidide beschrieben; sie beschreibt jedoch ebenfalls keine Thiophen-Verbindungen.

Die bekannten und auch die vorgeschlagenen Verbindungen entsprechen jedoch nicht allen gewünschten Anforderungen, so läßt ihre Wasserlöslichkeit noch zu wünschen übrig.

Außerdem wirken Sie noch nicht im gewünschten Maße selektiv. Es war daher wünschenswert, Verbindungen mit verbesserter Wasserlöslichkeit und Selektivität zu Verfügung zu stellen.

Dies ist durch die erfindungsgemäßen Verbindungen gelungen, welche keine unerwünschten und nachteiligen salidiuretischen, jedoch sehr gute antiarrhythmische Eigenschaften aufweisen, wie sie beispielsweise für die Behandlung von Krankheiten wichtig sind, die durch Sauerstoffmangel bewirkt werden. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei OrganTransplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlung von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferation von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwending werden.

### Liste der Abkürzungen:

- MeOH: Methanol
- DMF: N,N-Dimethylformamid
- El: electron impact
- DCI: Desorption-Chemical lonisation
- RT: Raumtemperatur
- EE: Ethylacetat (EtOAc)
- mp: Schmelzpunkt
- HEP: n-Heptan
- DME: Dimethoxyethan
- ES: Elektronenspray
- FAB: Fast Atom Bombardment
- CH₂Cl₂: Dichlormethan
- THF: Tetrahydrofuran
- eq.: Äquivalent

### Experimenteller Teil

### Allgemeine Vorschriften zur Herstellung von Alkenylcarbonsäure-guanididen (I)

Variante A: aus Alkenylcarbonsäuren (II, L = OH)
   1.0 eq. des Carbonsäurederivates der Formel II löst bzw. suspendiert man in wasserfreiem THF ( 5 ml/mmol) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck ( im Rotationsverdampfer) ab, versetzt mit Wasser, stellt mit 2 N HCI auf pH 6 bis 7 und filtriert das entsprechende Guanidid (Formel I) ab. Die so erhaltenen Carbonsäureguanidide können durch Behandeln mit wäßriger, methanolischer oder etherischer Salzsäure oder anderen pharmakologisch verträglichen Säuren in die entsprechenden Salze übergeführt werden.
Variante B: aus Alkenylcarbonsäure-alkylestern (II, L = O-Alkyl)
   1.0 eq. des Carbonsäure-alkylesters der Formel II sowie 5.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotationsverdampfer) abdestilliert, in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
   (Salzbildung vergleiche Variante A)

Beispiel 1: E-3-[2-(4-Methylsulfonyl-thiophenyl)]propensäureguanidid

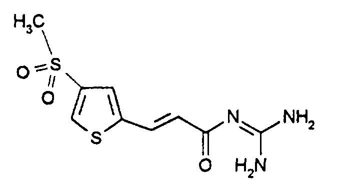

1 a) E-3-[2-(4-Methylsulfonyl-thiophenyl)]propensäuremethylester
1 eq. 3-[2-(4-Bromthiophenyl)]propensäuremethylester, 2 eq. Methylsulfinsäure-natriumsalz, 2 eq. Cul wurden in Toluol/DMF ( 2/3; 3 ml/mmol Ester) unter Rückfluß erhitzt. Standardaufarbeitung und Chromatographie an Kieselgel (Eluent: Cyclohexan/EE) ergab E-3-[2-(4-Methylsulfonyl-thiophenyl)]propensäuremethylester.
mp: amorph MS : 247 (M+1)⁺

1 b) Die Carbonsäure wurde unter Standardbedingungen (MeOH/NaOH) aus Ester 1 a) freigesetzt.
mp: 203°C MS :233 (M+1)⁺

1 c) 1 b wurde gemäß allgemeiner Vorschrift A ins Guanididhydrochlorid überführt.
mp: 200°C MS : 274 (M + 1) +

Beispiel 2: E-3-[2-(5-Methylthio-thiophenyl)]-2-methyl-propensäureguanidid Hydrochlorid

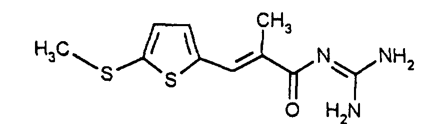

2 a) 1 eq. 2-Phosphonopropionsäuretriethylester wurde bei 0°C mit 1 eq. n-Butyllithium in Hexan deprotoniert und anschließend bei RT mit 1 eq. 5-Methylthiobenzaldehyd versetzt. Nach vollständiger Abreaktion des Aldehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte E-3-[2-(5-Methylthio-thiophenyl)]-2-methyl-propensäureethylester.
Farbloses Öl MS :243 (M+1)⁺

2 b) Der Ester aus 2 a) wurde gemäß Variante B ins zunächst ins Guanidid und anschließend ins Hydrochlorid überführt.
mp: 172°C MS :256 (M+1)+

Beispiel 3: E-3-[2-(5-Methylsulfonyl-thiophenyl)]-2-methyl-propensäureguanidid hydrochlorid

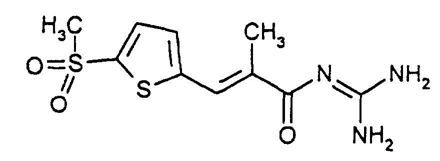

Der Ester aus 2 a) wurde gemäß einer Standardreaktion mit 2.2 eq m-Chlorperbenzoesäure in Methylenchlorid zum E-3-[2-(5-Methylsulfonyl-thiophenyl)]-2-methyl-propensäureethylester oxidiert.
Farbloses Öl MS : 275 (M+1)⁺

3 b) Der Ester aus 3 a) wurde zunächst mit Natriumhydroxid in Methanol in die freie Säure überführt und anschließend gemäß Variante A ins Guanididhydrochlorid.
Säure.
amorpher Feststoff MS: 247 (M+1)⁺

Guanididhydrochlorid:
mp: >210°C MS: 288 (M+1)⁺

Beispiel 4: E-3-[2-(3-Chlor-4-isopropylsulfonyl-5-methylthio-thiophenyl)]-2-methyl-propensäureguanidid hydrochlorid

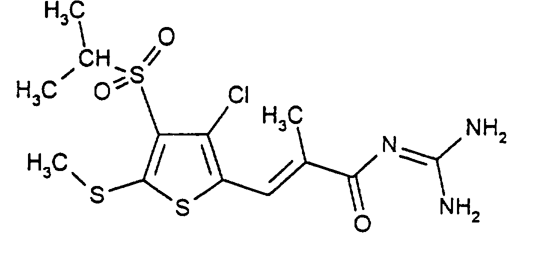

4 a) In Analogie zu 2 a) wurde der käufliche 3-Chlor-4-isopropylsulfonyl-5-methylthio-thiophenyl-2-carbaldehyd in den entsprechenden Propensäureester überführt.
Farbloses Öl MS :384 (M+1)⁺

4 b) Der Ester aus 4 a) wurde gemäß Variante B ins Guanidid überführt und als Hydrochlorid isoliert.
mp: 227 - 235°C MS : 396 (M+1)⁺

Beispiel 5: E-3-[2-(3-Chlor-4-isopropylsulfonyl-5-methylsulfonyl-thiophenyl)]-2-methyl-propensäureguanidid hydrochlorid

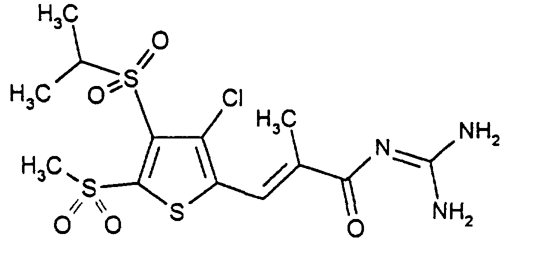

5 a) Der Ester aus 4 a) wurde mit einer Standardreaktion mit 2.2 eq m-Chlorperbenzoesäure in Methylenchlorid in den E-3-[2-(3-Chlor-4-isopropylsulfonyl-5-methylsulfonyl-thiophenyl)]-2-methyl-propensäureethylester überführt.
MS: 416 (M+1)⁺

5 b) Der Ester aus 5 a) wurde gemäß Variante B ins Guanidid überführt und als Hydrochlorid isoliert.
MS: 428 (M+1)⁺

## Patentansprüche

1. Substituierte Thiophenylalkenylcarbonsäureguanidide der Formel I 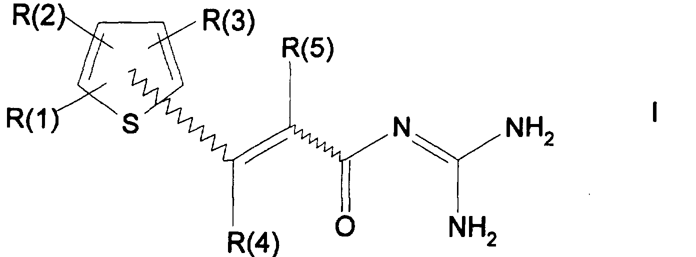 worin bedeuten:
mindestens einer der Substituenten R(1), R(2) und R(3)
R(31)SOₖ-;
k Null oder 2;
R(31) CH₃, CF₃ oder Phenyl,
das nicht substituiert oder substituiert ist mit 1 - 3 Substituenten ausgewählt aus der Gruppe bestehend aus F, Cl, CF₃, Methyl und Methoxy;
oder
R(31) NR(41)R(42); R(41) und R(42) unabhängig voneinander Wasserstoff, CH₃ oder CF₃;
oder
R(41) und R(42) gemeinsam 4 oder 5 Methylengruppen, von denen eine CH₂-Gruppe durch Sauerstoff, S, NH, N-CH₃ oder N-Benzyl ersetzt sein kann;
und die jeweils anderen Substituenten R(1), R(2) und R(3)
unabhängig voneinander H, F, Cl, CN, -Oₙₐ-CₘₐH_{2ma +1} oder -O_{ga}CᵣₐH₂ᵣₐR(10);
na Null oder 1;
ma Null, 1, 2, 3 oder 4;
ga Null oder 1;
ra Null oder 1;
R(10) Phenyl,
das nicht substituiert oder substituiert ist mit 1 - 3 Substituenten ausgewählt aus der Gruppe bestehend aus F, Cl, CF₃, Methyl und Methoxy;
R(4) und R(5)
unabhängig voneinander Wasserstoff, F, Cl, CH₃, CF₃;
sowie deren pharmazeutisch verträgliche Salze.

2. Verbindung der Formel I nach Anspruch 1, dadurch gekennzeichnet, daß sie ausgewählt ist aus der Gruppe bestehend aus
E-3-[2-(4-Methylsulfonyl-thiophenyl)]propensäureguanidid,
E-3-[2-(5-Methylthio-thiophenyl)]-2-methyl-propensäureguanidid,
E-3-[2-(5-Methylsulfonyl-thiophenyl)]-2-methyl-propensäureguanidid,
E-3-[2-(3-Chlor-4-isopropylsulfonyl-5-methylthio-thiophenyl)]-2-methyl-propensäureguanidid und
E-3-[2-(3-Chlor-4-isopropylsulfonyl-5-methylsulfonyl-thiophenyl)]-2-methyl-propensäureguanidid.

3. Verfahren zur Herstellung einer Verbindung der Formel I nach Anspruch 1, dadurch gekennzeichnet, daß man eine Verbindung der Formel II 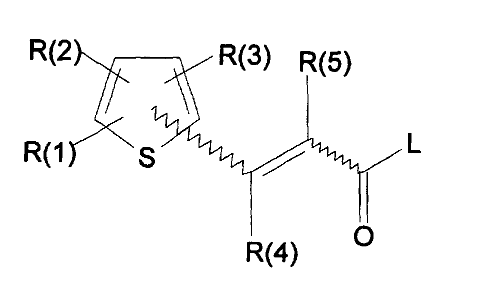 mit Guanidin umsetzt, worin R(1) bis R(5) die in Anspruch 1 angegebene Bedeutung besitzen und L für eine leicht nucleophil substituierbare leaving group steht.

4. Verwendung einer Verbindung I nach Anspruch I zur Herstellung eines Medikaments zur Behandlung oder Prophylaxe von durch ischämische Zustände hervorgerufenen Krankheiten.

5. Verwendung einer Verbindung 1 nach Anspruch 1 zur Herstellung eines Medikaments zur Behandlung von Arrhythmien.

6. Verwendung einer Verbindung I nach Anspruch 1 zur Herstellung eines Medikaments zur Behandlung oder Prophylaxe des Herzinfarkts.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch 1 zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I nach Anspruch 1 zur Herstellung eines Medikaments zur Behandlung oder Prophylaxe von ischämischen Zuständen peripherer Organe und Gliedmaßen.

11. Verwendung einer Verbindung I nach Anspruch 1 zur Herstellung eines Medikaments zur Behandlung von Schockzuständen.

12. Verwendung einer Verbindung 1 nach Anspruch 1 zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch 1 zur Herstellung eines Medikaments zur Behandlung von Krankheiten, bei denen die Zellproliferation eine primäre oder sekundäre Ursache darstellt.

15. Arzneimittel, enthaltend eine wirksame Menge einer Verbindung I nach einem oder mehreren der Ansprüche 1 oder 2.

## Claims

1. A substituted thiophenylalkenylcarboxylic acid guanidide of the formula I 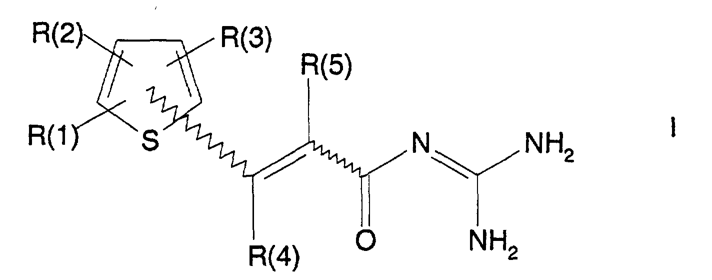 in which:
at least one of the substituents R(1), R(2) and R(3) is R(31)SOₖ-;
k is zero or 2;
R(31) is CH₃, CF₃ or phenyl,
which is unsubstituted or substituted by 1 - 3 substituents selected from the group consisting of F, Cl, CF₃, methyl and methoxy;
or
R(31) is NR(41)R(42);
R(41) and R(42) independently of one another are hydrogen, CH₃ or CF₃;
or
R(41) and R(42) together are 4 or 5 methylene groups, of which one CH₂ group can be replaced by oxygen, S, NH, N-CH₃ or N-benzyl;
and the other substituents R(1), R(2) and R(3) in each case
independently of one another are H, F, Cl, CN, -Oₙₐ-CₘₐH₂ₘₐ₊₁ or -O_{ga}CᵣₐH₂ᵣₐR(10);
na is zero or 1;
ma is zero, 1, 2, 3, or 4;
ga is zero or 1;
ra is zero or 1;
R(10) is phenyl,
which is unsubstituted or substituted by 1 - 3 substituents selected from the group consisting of F, Cl, CF₃, methyl and methoxy;
R(4) and R(5)
independently of one another are hydrogen, F, Cl, CH₃, CF₃;
and their pharmaceutically tolerable salts.

2. A compound of the formula I as claimed in claim 1, which is selected from the group consisting of
E-3-[2-(4-methylsulfonylthiophenyl)]propenoic acid guanidide,
E-3-[2-(5-methylthiothiophenyl)]-2-methyl-propenoic acid guanidide,
E-3-[2-(5-methylsulfonylthiophenyl)]-2-methyl-propenoic acid guanidide,
E-3-[2-(3-chloro-4-isopropylsulfonyl-5-methylthiothio-phenyl)]-2-methyl-propenoic acid guanidide and
E-3-[2-(3-chloro-4-isopropylsulfonyl-5-methylsulfonyl-thiophenyl)]-2-methylpropenoic acid guanidide.

3. A process for the preparation of a compound of the formula I as claimed in claim 1, which comprises reacting a compound of the formula II 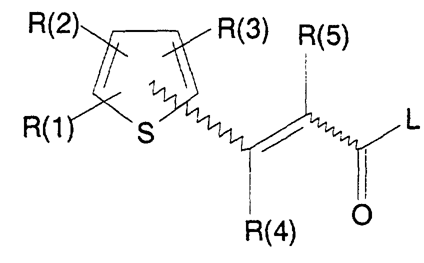 with guanidine, in which R(1) to R(5) have the meaning indicated in claim 1 and L is a leaving group which can be easily nucleophilically substituted.

4. The use of a compound 1 as claimed in Claim 1 for the production of a medicament for the treatment or prophylaxis of illnesses caused by ischemic conditions.

5. The use of a compound I as claimed in claim 1 for the production of a medicament for treatment of arrhythmias.

6. The use of a compound I as claimed in claim 1 for the production of a medicament for the treatment or prophylaxis of myocardial infarction.

7. The use of a compound I as claimed in claim 1 for the production of a medicament for the treatment or prophylaxis of angina pectoris.

8. The use of a compound I as claimed in claim 1 for the production of a medicament for the treatment or prophylaxis of ischemic conditions of the heart.

9. The use of a compound I as claimed in claim 1 for the production of a medicament for the treatment or prophylaxis of ischemic conditions of the peripheral and central nervous system and stroke.

10. The use of a compound I as claimed in claim 1 for the production of a medicament for the treatment or prophylaxis of ischemic conditions of peripheral organs and limbs.

11. The use of a compound I as claimed in claim 1 for the production of a medicament for the treatment of states of shock.

12. The use of a compound I as claimed in claim 1 for the production of a medicament for use in surgical operations and organ transplantations.

13. The use of a compound I as claimed in claim 1 for the production of a medicament for the preservation and storage of transplants for surgical measures.

14. The use of a compound I as claimed in claim 1 for the production of a medicament for the treatment of illnesses in which cell proliferation is a primary or secondary cause.

15. A pharmaceutical comprising an efficacious amount of a compound I as claimed in one or more of claims 1 and 2.

## Revendications

1. Guanidides d'acides thiophénylalkénylcarboxyliques substitués de formule I 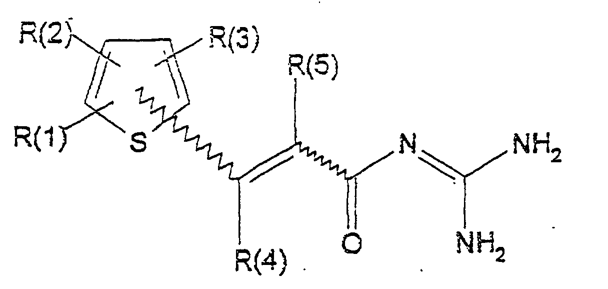 dans laquelle :
au moins l'un des substituants R(1), R(2) et R(3) représente
R(31)SOₖ- ;
k représente zéro ou 2 ;
R(31) représente CH₃, CF₃ ou phényle
qui n'est pas substitué ou est substitué par 1 à 3 substituants choisis dans le groupe constitué de F, C1, CF₃, méthyle et méthoxy ;
ou
R(31) représente NR(41)R(42) ;
R(41) et R(42), indépendamment l'un de l'autre, représentent l'hydrogène, CH₃ ou CF₃ ;
ou
R(41) et R(42) représentent
ensemble 4 ou 5 groupes méthylène, dont un groupe CH₂ peut être remplacé par l'oxygène, S, NH, N-CH₃ ou N-benzyle ;
et les autres substituants respectifs R(1), R(2) et R(3) représentent,
indépendamment l'un de l'autre, H, F, Cl, CN, -Oₙₐ-CₘₐH₂ₘₐ₊₁ ou -O_{ga}CᵣₐH₂ᵣₐR(10) ;
na représente zéro ou 1 ;
ma représente zéro ou 1, 2, 3 ou 4 ;
ga représente zéro ou 1 ;
ra représente zéro ou 1 ;
R(10) représente phényle
qui n'est pas substitué ou est substitué par 1 à 3 substituants choisis dans le groupe constitué de F, Cl, CF₃, méthyle et méthoxy ;
R(4) et R(5) représentent,
indépendamment l'un de l'autre, l'hydrogène, F, Cl, CH₃, CF₃ ;
ainsi que leurs sels pharmaceutiquement compatibles.

2. Composé de formule I selon la revendication 1, caractérisé en ce qu'il est choisi dans le groupe constitué du
guanidide de l'acide
E-3-[2-(4-méthylsulfonyl-thiophényl)]propènoïque,
guanidide de l'acide
E-3-[2-(5-méthylthio-thiophényl]-2-méthyl-propènoïque,
guanidide de l'acide
E-3-[2-(5-méthylsulfonyl-thiophényl]-2-méthyl-propènoïque,
guanidide de l'acide
E-3-[2-(3-chloro-4-isopropylsulfonyl-5-méthylthio-thiophényl)]-2-méthyl-propènoïque et
guanidide de l'acide
E-3-[2-(3-chloro-4-isopropylsulfonyl-5-méthylsulfonyl-thiophényl)]-2-méthyl-propènoïque.

3. Procédé pour la préparation d'un composé de formule I selon la revendication 1, caractérisé en ce que
on fait réagir un composé de formule II 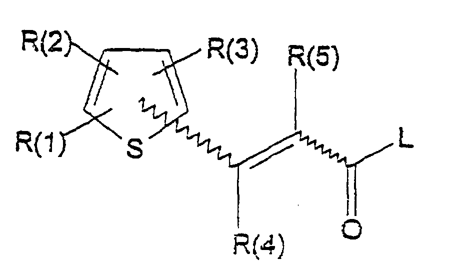 avec la guanidine, composé dans lequel R(1) à R(5) possèdent la signification donnée à la revendication 1 et L représente un groupe partant facilement substituable de façon nucléophile.

4. Utilisation d'un composé I selon la revendication 1 pour la préparation d'un médicament pour le traitement ou la prophylaxie de maladies provoquées par des états ischémiques.

5. Utilisation d'un composé I selon la revendication 1 pour la préparation d'un médicament pour le traitement des arythmies.

6. Utilisation d'un composé I selon la revendication 1 pour la préparation d'un médicament pour le traitement ou la prophylaxie de l'infarctus cardiaque.

7. Utilisation d'un composé I selon la revendication 1 pour la préparation d'un médicament pour le traitement ou la prophylaxie de l'angine de poitrine.

8. Utilisation d'un composé I selon la revendication 1 pour la préparation d'un médicament pour le traitement ou la prophylaxie des états ischémiques du coeur.

9. Utilisation d'un composé I selon la revendication 1 pour la préparation d'un médicament pour le traitement ou la prophylaxie des états ischémiques du système nerveux périphérique et central et de l'attaque d'apoplexie.

10. utilisation d'un composé I selon la revendication 1 pour la préparation d'un médicament pour le traitement ou la prophylaxie des états ischémiques des organes périphériques et des membres.

11. utilisation d'un composé I selon la revendication 1 pour la préparation d'un médicament pour le traitement des états de choc.

12. Utilisation d'un composé I selon la revendication 1 pour la préparation d'un médicament à utiliser lors des opérations chirurgicales et des transplantations d'organes.

13. Utilisation d'un composé I selon la revendication 1 pour la préparation d'un médicament pour la conservation et l'entreposage de transplants pour des prises de mesures chirurgicales.

14. Utilisation d'un composé I selon la revendication 1 pour la préparation d'un médicament pour le traitement de maladies pour lesquelles la prolifération cellulaire représente une cause primaire ou secondaire.

15. Médicament contenant une quantité efficace d'un composé I selon l'une ou plusieurs des revendications 1 ou 2.
